# EUROPÄISCHE PATENTSCHRIFT

(11) **EP 0 718 292 B1**
(45) Veröffentlichungstag und Bekanntmachung des Hinweises auf die Patenterteilung: **22.04.1998**
(21) Anmeldenummer: 96101050.1
(22) Anmeldetag: 31.07.1993
(51) Int. Cl.: C07D 261/08, A01N 43/80, C07C 69/734, C07C 69/738, A01N 37/10

(54) **Acetylenderivate und diese enthaltende Pflanzenschutzmittel**
Acetylenic derivatives and their use as plant-protective agents
Dérivés acétyléniques et leur application comme agents phytosanitaires

(30) Priorität: 11.08.1992 DE 4226557; 27.11.1992 DE 4239874
(43) Veröffentlichungstag der Anmeldung: 26.06.1996
(62) Teilanmeldung aus: 93112327.7
(73) Patentinhaber: BASF AKTIENGESELLSCHAFT, 67056 Ludwigshafen (DE)
(72) Erfinder: Wingert, Horst, Dr., D-68159 Mannheim (DE); Hellendahl, Beate, Dr., D-67105 Schifferstadt (DE); Kirstgen, Reinhard, Dr., D-67434 Neustadt (DE); Sauter, Hubert, Dr., D-68167 Mannheim (DE); Ammermann, Eberhard, Dr., D-64646 Heppenheim (DE); Lorenz, Gisela, Dr., D-67434 Hambach (DE)

(56) Entgegenhaltungen:
- EP-A- 0 178 826
- EP-A- 0 253 213
- EP-A- 0 280 185
- EP-A- 0 469 411
- EP-A- 0 477 631

## Beschreibung

Die vorliegende Erfindung betrifft neue Acetylenderivate und ein Verfahren zur Bekämpfung von Schädlingen, insbesondere von Pilzen, Insekten, Nematoden und Spinnmilben mit diesen Verbindungen.

Es ist bekannt, daß einzelne Acetylenderivate (EP-A 178 826, EP-A 244 077, EP-A 253 213, EP-A 280 185, EP-A 477 631) fungizide oder insektizide Wirkung besitzen. Ihre Wirkung ist jedoch unbefriedigend.

Es wurde nun überraschend gefunden, daß die neuen Acetylenderivate der allgemeinen Formel I 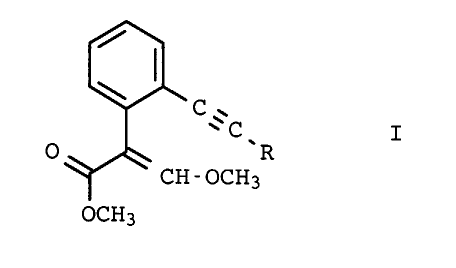 in der
- R: Phenyl bedeutet, welches in 4-Stellung durch Chlor substituiert und ggf. einen weiteren Substituenten aus der Gruppe: 2-Chlor, 3-Chlor, 2-Nitro, 3-Trifluormethyl oder
in 4-Stellung durch Fluor und ggf. zusätzlich durch 2-Methyl substituiert oder
in 4-Stellung durch Brom oder Jod substituiert ist
eine ausgezeichnete fungizide, insektizide, nematizide und akarizide Wirkung haben.

Die fungizide und insektizide Wirkung wird bevorzugt.

Die Herstellung der Verbindungen der allgemeinen Formel I erfolgt beispielsweise wie in den folgenden Schemata beschrieben.

Die neuen Verbindungen der allgemeinen Formel I können hergestellt werden, indem man beispielsweise eine o-Brom-Verbindung 2 mit einem terminalen Acetylenderivat 3 in Gegenwart eines Übergangsmetallkatalysators, vorzugsweise in Gegenwart einer Palladiumverbindung, wie z.B. Pd(PPh₃)₄, PdCl₂, Pd(OAc)₂, in Gegenwart von Triphenylphosphin und in Gegenwart einer Base, wie z.B. in Gegenwart eines N-Alkylamins, wie beispielsweise Triethylamin, ggf. in Gegenwart eines Lösungs- oder Verdünnungsmittels, wie z.B. DMF oder Acetonitril umsetzt [vgl. z.B. L. Brandsma et al., Synth. Comm. 20, 1889 (1990); W. B. Austin et al., J. Org. Chem. 46, 2280 (1981)]. 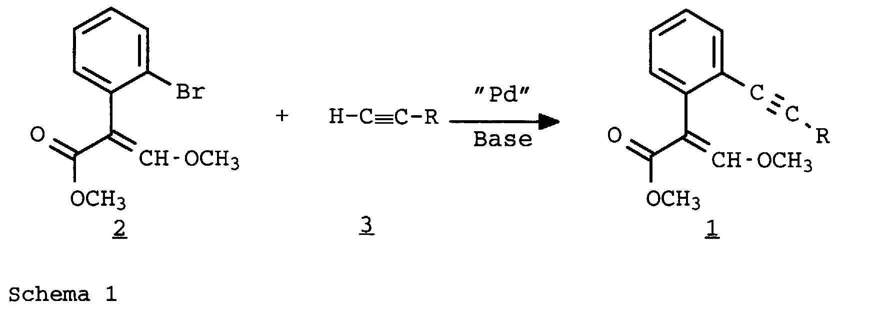 Alternativ hierzu erhält man die Verbindungen der allgemeinen Formel 1, indem man die o-Brom-a-Ketosäurederivate 4 mit den Acetylenen der Formel 3 umsetzt (Bedingungen analog Schema 1) 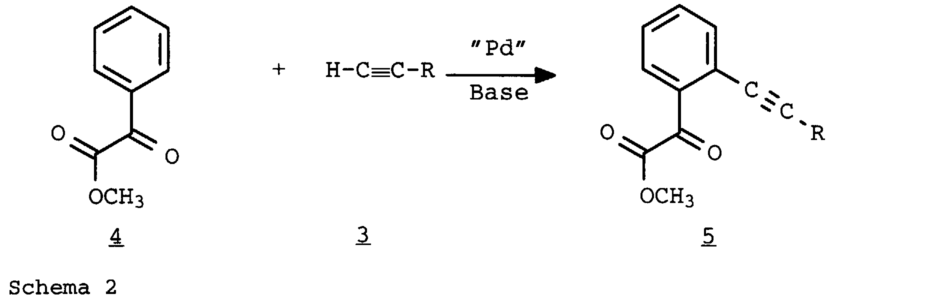 und anschließend die erhaltenen a-Ketocarbonsäurederivate 5 zum Beispiel im Sinne einer Wittig- bzw. im Sinne einer Wittig-Horner-Emmons-Reaktion mit einem Phosphoniumylid 6 bzw. mit einem Phosphonsäurederivat 7 (Schema 3) zu den neuen Verbindungen der Formel 1 umsetzt (vgl. z. B. C. Ferri; Reaktionen der organischen Synthese, Thieme Verlag, Stuttgart, S. 354 ff, 1978).

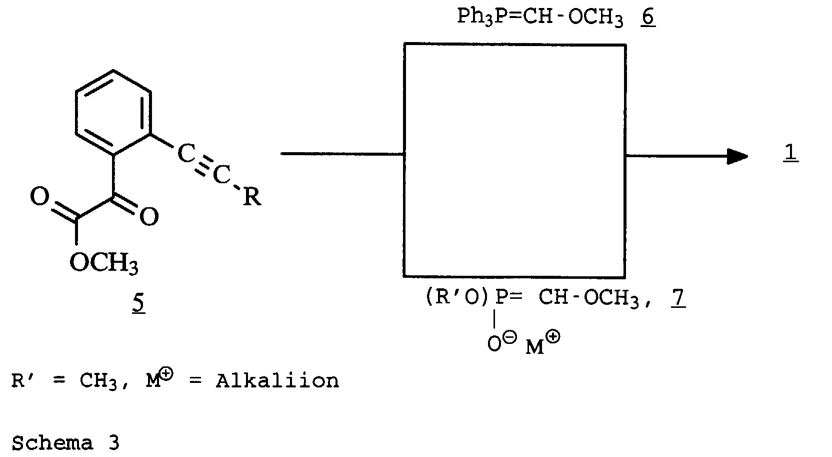

Die neuen Verbindungen der allgemeinen Formel 1 lassen sich auch herstellen, indem man die terminalen Acetylene 9 im Sinne einer Übergangsmetall(ÜM)-katalysierten Kupplung mit den Halogeniden 10, vorzugsweise mit den Bromiden (X = Br) bzw. den Jodiden (X = J) in Gegenwart eines Katalysators, wie z. B. PdCl₂, Pd(OAc)₂ oder Pd(PPh₃)₄ in Gegenwart einer Base, wie etwa Triethylamin zu Reaktion bringt (vgl. z. B. P.C. Vollhardt, Angew. Chem. 98, 268 (1986), H. Yamanaka, Synthesis, 312 (1983)).

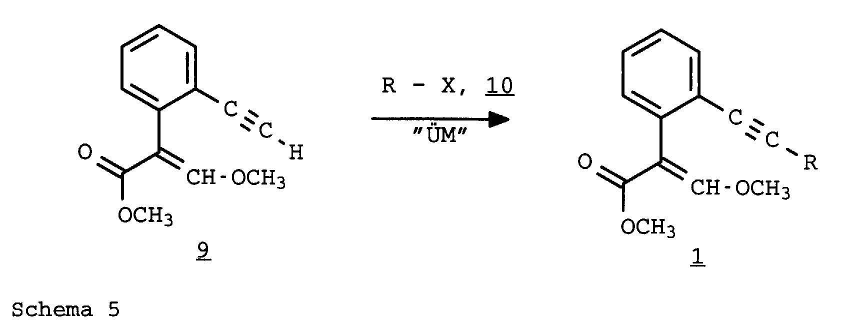

Alternativ hierzu kann man auch so vorgehen, daß man die α-Ketosäurederivate der terminalen Acetylene 11 mit den Halogeniden 10 übergangsmetall (ÜM) katalysiert in die Derivate 5 überführt und diese wie zuvor beschrieben in die neuen Verbindungen der Formel 1 überführt.

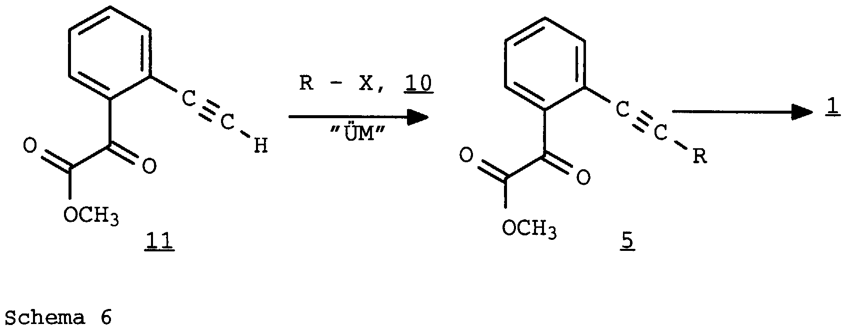

Eine weitere Möglichkeit zur Darstellung der Verbindungen der allgemeinen Formel 1 besteht darin, daß man zuerst die Acetylengruppierung in Formel 12, nach bekannten Methoden (vgl. z. B. Houben-Weyl, Bd. V/2a, S. 33 f Viehe, bzw. siehe Schema 1 + 2) aufbaut, 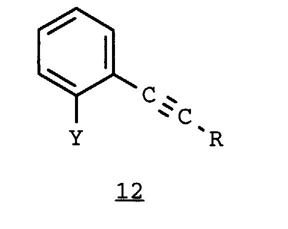 und anschließend die Gruppe Y in die Gruppierung 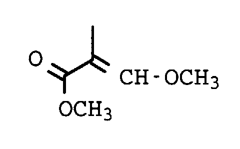 überführt.

Schemata 7-9 sollen dies verdeutlichen. Die Ketocarbonsäureester 5 lassen sich ausgehend von den o-Brombenzaldehyden 13 darstellen.

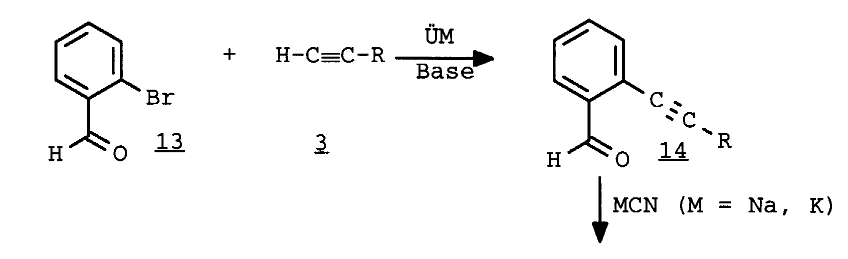

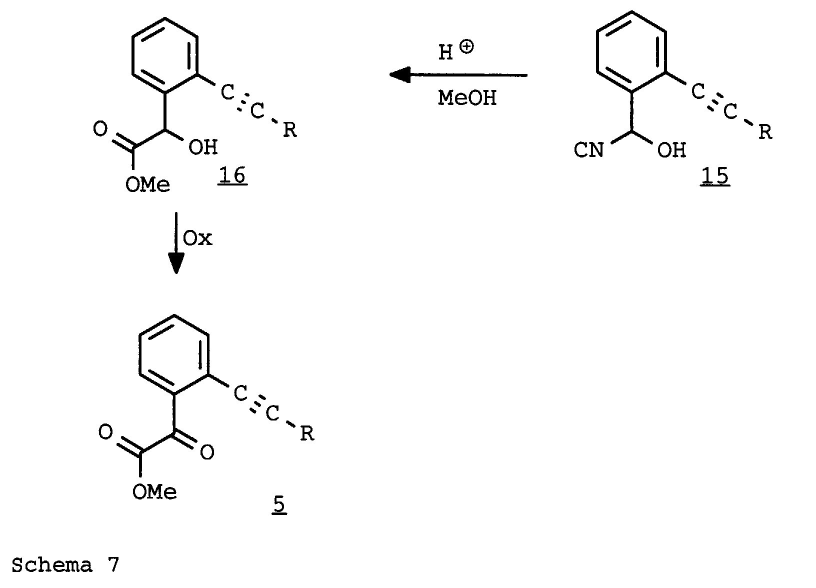

Die o-Brom-benzaldehyde 12 lassen sich mit den terminalen Acetylenen 3 Übergangsmetall-katalysiert zu den Aldehyden 14 umsetzen (vgl. W.B. Austin, J. Org. Chem., 46 2280, (1981)).

Aus den Aldehyden 14 erhält man die Cyanhydrine 15 durch Umsetzung mit einem Metallcyanid, wie NaCN oder KCN in Gegenwart einer Säure, z. B. Salzsäure (vgl. z. B. Organikum, 16. Auflage, S. 445 (1986)).

Über eine "Pinner-Reaktion" lassen sich aus den Cyanhydrinen 15 die Mandelsäurederivate 16 darstellen (vgl. z.B. Org. Synth. Coll. Vol. 4, 58 (1963)).

Die Oxidation der Mandelsäurederivate 16, beispielsweise mit Chromverbindungen, wie Chromtrioxid, mit Natriumhypochlorid oder mit TEMPO liefert die α-Ketoester 5 (vgl. z. B. Houben-Weyl, Bd. 4/lb, S. 425-464 (1975) (TEMPO = Tetramethylpiperidin-1-oxyl).

Die a-Ketocarbonsäureester 5 lassen sich auch ausgehend von den Halogenderivaten 18 durch Grignard-Reaktion mit Oxalsäureverbindungen 19 analog Schema 8 aufbauen (vgl. z. B. J.S. Nimitz, J. Org. Chem., 46, 211 (1981). 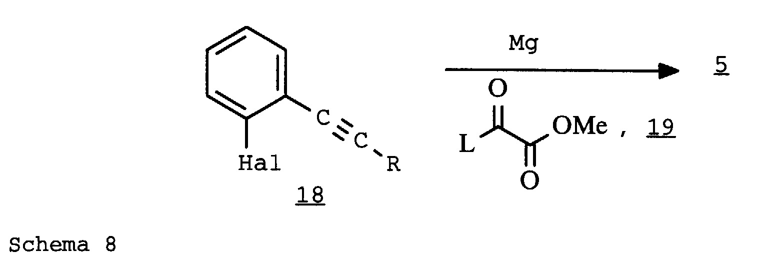 Eine weitere Möglichkeit besteht darin, Carbonsäuren 20 über die Stufe der Säurechloride in die Cyanide 21 zu überführen und diese im Sinne einer Pinnerreaktion zu den Ketoestern 5 umzuwandeln. 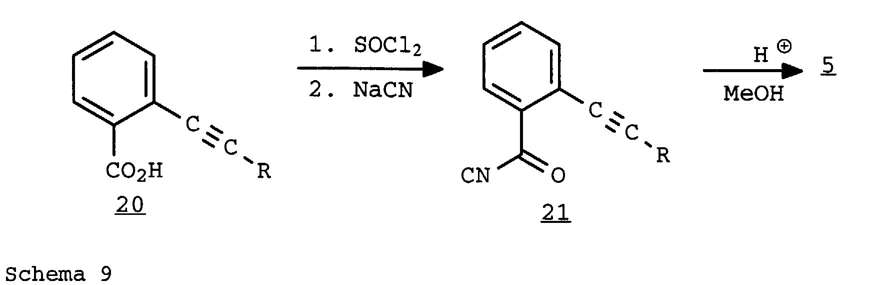 (vgl. z. B. Organikum, 16. Auflage, 423 f (1986), Angew. Chem. 94, 1, 1982)).

Die neuen Verbindungen der allgemeinen Formel I lassen sich aber auch so darstellen, daß man eine geeignet substituierte Styryl-Verbindung 25 in an sich bekannter Weise in das entsprechende Acetylenderivat 1 überführt.

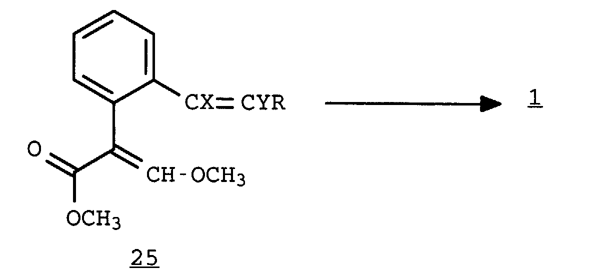

Für den Fall, daß X = H und Y = Halogen bzw. X = Halogen und Y = H, wird die Reaktion in Gegenwart einer Base wie z. B. Natriumhydrid, Kaliumhydroxid, Triton B oder n-Butyllithium durchgeführt (vgl. z. B. H. Zimmer et al, Chimia 28 (1974), S. 656 f) (Triton B = 2-Benzyltrimethylammoniumhydroxid). Im Falle von X und Y = Halogen läßt sich die Abspaltung mit Zink durchführen (vgl. z. B. H.G. Viehe, Chemistry of Acetylenes, Marcel Dekker, New York, 1969, S. 134 f).

### Beispiele

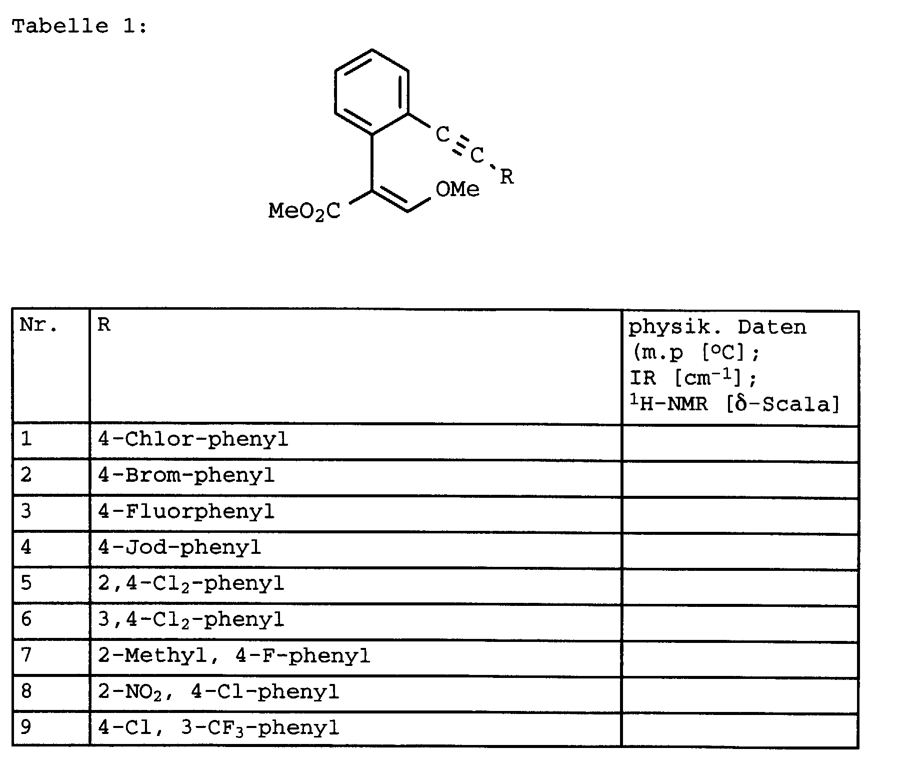

Die neuen Verbindungen eignen sich als Fungizide.

Die erfindungsgemäßen fungiziden Verbindungen bzw. die sie enthaltenden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungformen richten sich nach den Verwendungszwecken; sie sollten in jedem Fall möglichst die feinste Verteilung der erfindungsgemäßen Wirkstoffe gewährleisten. Normalerweise werden die Pflanzen mit den Wirkstoffen besprüht oder bestäubt oder die Samen der Pflanzen mit den Wirkstoffen behandelt.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Ligninsulfitablaugen und Methylcellulose.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Wirkstoffe zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Die Verbindungen werden angewendet, indem man die Pilze oder die vor Pilzbefall zu schützenden Saatgüter, Pflanzen, Materialien oder den Erdboden mit einer fungizid wirksamen Menge der Wirkstoffe behandelt.

Die Anwendung erfolgt vor oder nach der Infektion der Materialien, Pflanzen oder Samen durch die Pilze.

Speziell eignen sich die Verbindungen I zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die neuen Verbindungen können auch im Materialschutz (Holzschutz) eingesetzt werden, z.B. gegen Paecilomyces variotii.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff pro ha.

Bei der Saatgutbehandlung werden im allgemeinen Wirkstoffmengen von 0,001 bis 50 g, vorzugsweise 0,01 bis 10 g je Kilogramm Saatgut benötigt.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln.

Beim Vermischen mit Fungiziden erhält man dabei in vielen Fällen eine Vergrößerung des fungiziden Wirkungsspektrums.

## Patentansprüche

1. Acetylenderivate der allgemeinen Formel I 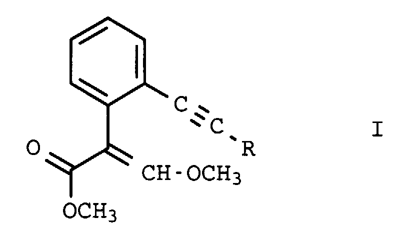 in der
R Phenyl bedeutet, welches in 4-Stellung durch Chlor substituiert und ggf. einen weiteren Substituenten aus der Gruppe: 2-Chlor, 3-Chlor, 2-Nitro, 3-Trifluormethyl oder
in 4-Stellung durch Fluor und ggf. zusätzlich durch 2-Methyl substituiert oder
in 4-Stellung durch Brom oder Jod substituiert ist.

2. Fungizid, enthaltend einen inerten Trägerstoff und eine fungizid wirksame Menge eines Acetylenderivats der Formel I gemäß Anspruch 1.

3. Verfahren zur Bekämpfung von Pilzen dadurch gekennzeichnet, daß man die Pilze oder die von Pilzbefall bedrohten Materialien, Pflanzen, Saatgut oder den Erdboden mit einer fungizid wirksamen Menge eines Acetylenderivats der Formel I gemäß Anspruch 1 behandelt.

4. Verfahren zur Bekämpfung von Insekten, Nematoden und Spinnmilben, dadurch gekennzeichnet, daß man diese oder den Ort, an dem sie sich aufhalten, mit einer wirksamen Menge eines Acetylenderivats der Formel I gemäß Anspruch 1 behandelt.

## Claims

1. An acetylene derivative of the formula I 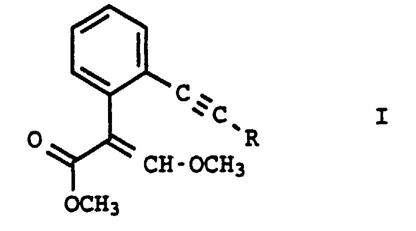 where
R is phenyl which is substituted in position 4 by chlorine and which may be substituted by a further substituent from the group consisting of: 2-chloro, 3-chloro, 2-nitro, 3-trifluoromethyl or
is substituted in position 4 by fluorine and which may additionally be substituted by 2-methyl or
is substituted in position 4 by bromine or iodine.

2. A fungicide containing an inert carrier and a fungicidal amount of an acetylene derivative of the formula I as claimed in claim 1.

3. A method for controlling fungi, wherein the fungi or the materials, plants or seed threatened by fungal attack or the soil is or are treated with a fungicidal amount of an acetylene derivative of the formula I as claimed in claim 1.

4. A method for controlling insects, nematodes and spider mites, wherein these or the place where they are present is or are treated with an effective amount of an acetylene derivative of the formula I as claimed in claim 1.

## Revendications

1. Dérivés acétyléniques de formule générale I 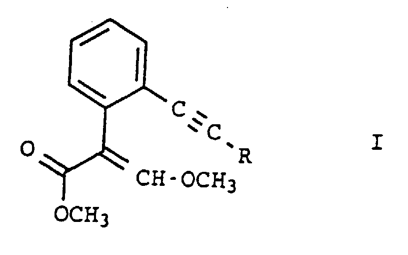 dans laquelle
R représente un groupe phényle substitué par le chlore en position 4 et portant éventuellement un autre substituant du groupe : 2-chloro, 3-chloro, 2-nitro, 3-trifluorométhyle, ou bien
substitué par le fluor en position 4 et portant le cas échéant un substituant 2-méthyle, ou bien
substitué par le brome ou l'iode en position 4.

2. Produit fongicide contenant un véhicule inerte et une quantité fongicide efficace d'un dérivé acétylénique de formule I selon la revendication 1.

3. Procédé pour combattre les mycètes, caractérisé par le fait que l'on traite les mycètes ou les matières, végétaux, semences ou le sol menacés d'une attaque par les mycètes par une quantité fongicide efficace d'un dérivé acétylénique de formule I selon la revendication 1.

4. Procédé pour combattre les insectes, les nématodes et les acariens, caractérisé par le fait qu'on les traite ou on traite l'endroit où ils séjournent par une quantité efficace d'un dérivé acétylénique de formule I selon la revendication 1.
